# EUROPEAN PATENT APPLICATION

(11) **EP 4 722 204 A1**
(43) Date of publication of application: **08.04.2026**
(21) Application number: 24818608.2
(22) Date of filing: 03.06.2024
(51) Int. Cl.: C07D 401/10

(54) **INDUSTRIAL PREPARATION OF HETEROCYCLIC MODULATOR FOR LIPID SYNTHESIS**

(30) Priority: 05.06.2023 CN 202310658687
(71) Applicant: Sagimet Biosciences Inc., San Mateo, California 94402 (US)
(72) Inventor: WU, Jinzi Jason, Shanghai 200131 (CN); WAGMAN, Allan, San Mateo, California 94402 (US)
(74) Representative: Bassil, Nicholas Charles
(86) International application number: PCT/CN2024/097023
(87) International publication number: WO 2024/251076

(57) **Abstract**

Disclosed is a method for preparing a compound SGL1, comprising: subjecting a compound SGL1-SM1 to a hydrolysis reaction under an alkaline condition, to obtain a compound SGL1-A; subjecting the compound SGL1-A to a reaction with dimethylaminoacetaldehyde dimethylacetal and hydrazine acetate to obtain a compound SGL1-B; subjecting the compound SGL1-B to a hydrolysis reaction under an alkaline condition to obtain a compound SGL1-C; and subjecting the compound SGL1-C to a reaction with a compound SGL1-SM2 to obtain the compound SGL1.

## Description

### Reference to Related Application

The present disclosure claims all rights and interests of the patent application for invention with the application number 202310658687.7, titled "INDUSTRIAL PREPARATION OF HETEROCYCLIC MODULATOR FOR LIPID SYNTHESIS," and filed with the National Intellectual Property Administration of the People's Republic of China on 5 June 2023, the entire content of which is incorporated into the present disclosure by reference.

### Field

The present disclosure relates generally to the field of pharmaceutical chemistry, and more specifically relates to the field of pharmaceutical synthesis.

### Background

A compound SGL1, also known as ASC40 (foreign code: TVB-2640), is a powerful and safe oral small molecule inhibitor of selective fatty acid synthase, and has a chemical name: 4-(1-(4-cyclobutyl-2-methyl-5-(5-methyl-4H-1,2,4-triazol-3-yl)benzoyl)piperidin-4-yl)benzonitrile, a molecular formula: C₂₇H₃₁N₅O₂, and a structural formula as follows:

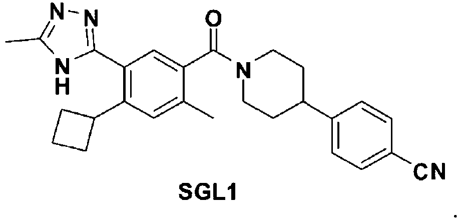

### Summary

In a first aspect, the present disclosure relates to a method for preparing a compound SGL1-A, comprising: subjecting a compound SGL1-SM1 to a hydrolysis reaction under an alkaline condition, wherein an aqueous solution of hydrogen peroxide is added to the hydrolysis reaction.

In another aspect, the present disclosure relates to a method for preparing a compound SGL1-B, comprising: subjecting a compound SGL1-A to a reaction with dimethylaminoacetaldehyde dimethylacetal and hydrazine acetate.

In still another aspect, the present disclosure relates to a method for preparing a compound SGL1-C, comprising: subjecting a compound SGL1-B to a hydrolysis reaction under an alkaline condition.

In yet another aspect, the present disclosure relates to a method for preparing a compound SGL1, comprising: subjecting a compound SGL1-C to a reaction with a compound SGL1-SM2.

In another aspect, the present disclosure relates to a method for preparing a compound SGL1, comprising: the method for preparing a compound SGL1-A according to the present disclosure; and/or the method for preparing a compound SGL1-B according to the present disclosure; and/or the method for preparing a compound SGL1-C according to the present disclosure; and/or the method for preparing a compound SGL1 according to the present disclosure.

In still another aspect, the present disclosure relates to the method for preparing a compound SGL1, comprising: subjecting a compound SGL1-SM1 to a hydrolysis reaction under an alkaline condition to obtain a compound SGL1-A; subjecting the compound SGL1-A to a reaction with dimethylaminoacetaldehyde dimethylacetal and hydrazine acetate to obtain a compound SGL1-B; subjecting the compound SGL1-B to a hydrolysis reaction under an alkaline condition to obtain a compound SGL1-C; and subjecting the compound SGL1-C to a reaction with a compound SGL1-SM2 to obtain the compound SGL1.

### Details

In the following description, some specific details are included to provide thorough understanding of various disclosed embodiments. However, those skilled in the relevant art will recognize that the embodiments may still be implemented using other methods, components, materials, etc. without using one or more of these specific details.

Unless otherwise required in the present application, throughout the specification and the appended claims, the words "comprising," "including," "containing," and "having" should be construed as an open-ended and inclusive meaning, i.e., "including but not limited to."

As used in the present disclosure and the appended claims, a singular referent without an indication of the number includes plural referents unless the context explicitly dictates otherwise.

Reference throughout the specification to "one embodiment," "an embodiment," "in another embodiment," or "in some embodiments" means that in at least one embodiment, a specific reference element, structure, or feature associated with the embodiment is included. Therefore, appearance of the wording "in one embodiment," "in an embodiment," "in another embodiment," or "in some embodiments" at different positions throughout the specification does not necessarily refer to a same embodiment. In addition, specific elements, structures, or features may be combined in any suitable manner in one or more embodiments.

It should be understood that, as used in the specification and the appended claims of the present disclosure, the article "one" (corresponding to "a", "an" and "the" in English) in a singular form comprises plural objects unless the context explicitly dictates otherwise. Therefore, for example, reference to "adding an acid to a reaction of a compound SGL1-A with dimethylaminoacetaldehyde dimethylacetal and hydrazine acetate" includes "adding an acid to a reaction of the compound SGL1-A with dimethylaminoacetaldehyde dimethylacetal and hydrazine acetate", or "adding two or more acids to a reaction of the compound SGL1-A with dimethylaminoacetaldehyde dimethylacetal and hydrazine acetate."

### Definitions

Therefore, unless otherwise stated, the following terms used in the specification and the appended claims have the following meanings:
In the present disclosure, the term "compound SGL1-A" refers to 5-carbamoyl-4-cyclobutyl-2-methylbenzoic acid, which has the following structure:

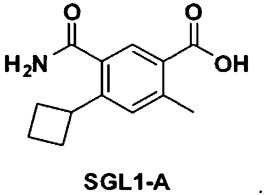

In the present disclosure, the term "compound SGL1-B" refers to methyl 4-cyclobutyl-2-methyl-5-(5-methyl-4H-1,2,4-triazol-3-yl)benzoate·hydrochloride, which has the following structure:

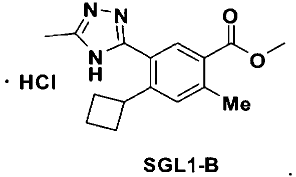

In the present disclosure, the term "compound SGL1-C" refers to 4-cyclobutyl-2-methyl-5-(5-methyl-4H-1,2,4-triazol-3-yl)benzoic acid, which has the following structure:

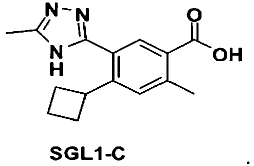

In the present disclosure, the term "compound SGL1-SM1" refers to 5-cyano-4-cyclobutyl-2-methylbenzoic acid, which has the following structure:

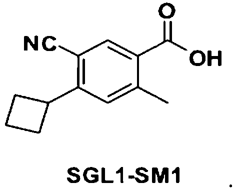

In the present disclosure, the term "compound SGL1-SM2" refers to 4-(piperidin-4-yl) benzonitrile, which has the following structure:

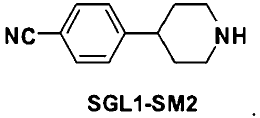

In the present disclosure, the term "compound SGL1" refers to 4-(1-(4-cyclobutyl-2-methyl-5-(5-methyl-4H-1,2,4-triazol-3-yl)benzoyl)piperidin-4-yl)benzonitrile, which has the following structure:

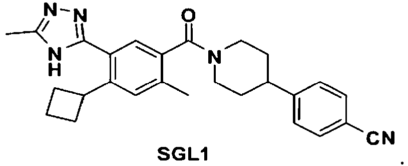

In the present disclosure, the term "equivalent" refers to a mass ratio of substances when they interact, and is calculated based on specific reactants specified.

In the present disclosure, the term "condensation agent" refers to a reaction adjuvant added in a condensation reaction.

### Detailed Description

In a first aspect, the present disclosure relates to a method for preparing a compound SGL1-A, comprising: subjecting a compound SGL1-SM1 to a hydrolysis reaction under an alkaline condition, wherein an aqueous solution of hydrogen peroxide is added to the hydrolysis reaction.

In some embodiments, an aqueous solution of hydrogen peroxide that can be used in the present disclosure has a concentration of about 20-70% by mass.

In some embodiments, an addition amount of the aqueous solution of hydrogen peroxide that can be used in the present disclosure is about 7-15 equivalents based on the compound SGL1-SM1.

In some embodiments, the hydrolysis reaction is carried out at about 20-30°C.

In some embodiments, the hydrolysis reaction is carried out for about 5-10 h.

In some embodiments, illustrative examples of the alkaline condition that can be used in the method for preparing a compound SGL1-A of the present disclosure include, but are not limited to, sodium hydroxide, potassium hydroxide, lithium hydroxide, potassium carbonate, cesium carbonate, sodium carbonate, or any mixture thereof.

In some embodiments, because of having oxidizing ability, hydrogen peroxide can oxidize easily oxidizable cyano into a more stable thiocyanate ion (SCN-), thereby preventing the cyano from being hydrolyzed into an acid, inhibiting the production of by-products, and improving the yield.

In some embodiments, the method for preparing a compound SGL1-A of the present disclosure can reach 10 Kg level scale-up yield of more than 90%.

In another aspect, the present disclosure relates to a method for preparing a compound SGL1-B, comprising: subjecting a compound SGL1-A to a reaction with dimethylaminoacetaldehyde dimethylacetal and hydrazine acetate.

In some embodiments, an addition amount of dimethylaminoacetaldehyde dimethylacetal is about 3.0-6.0 equivalents based on the compound SGL1-A.

In some embodiments, an addition amount of hydrazine acetate is 1.5-3.0 equivalents based on the compound SGL1-A.

In some embodiments, the reaction is carried out at about 60-70°C.

In some embodiments, a product of subjecting the compound SGL1-A to a reaction with dimethylaminoacetaldehyde dimethylacetal and hydrazine acetate in the presence of an organic solvent is refined.

In some embodiments, illustrative examples of the organic solvent that can be used to refine the product of subjecting the compound SGL1-A to the reaction with dimethylaminoacetaldehyde dimethylacetal and hydrazine acetate include, but are not limited to, methanol, ethanol, isopropanol, methyl tert-butyl ether, n-heptane, isopropyl acetate, acetone, methyl isobutyl ketone, isobutyl acetate, ethyl acetate, or any mixture thereof.

In some embodiments, the method for preparing a compound SGL1-B further comprises refining a product of subjecting a compound SGL1-A to a reaction with dimethylaminoacetaldehyde dimethylacetal and hydrazine acetate in the presence of ethyl acetate.

In some embodiments, the method for preparing a compound SGL1-B further comprises adding an acid to the reaction of the compound SGL1-A with dimethylaminoacetaldehyde dimethylacetal and hydrazine acetate.

In some embodiments, illustrative examples of the acid that can be used in the reaction of the compound SGL1-A with dimethylaminoacetaldehyde dimethylacetal and hydrazine acetate include, but are not limited to, formic acid, acetic acid, phosphoric acid, hydrochloric acid, maleic acid, fumaric acid, or any mixture thereof.

In some embodiments, the method for preparing a compound SGL1-B further comprises adding acetic acid to the reaction of the compound SGL1-A with dimethylaminoacetaldehyde dimethylacetal and hydrazine acetate.

In some embodiments, an amount of the acid added to the reaction of the compound SGL1-A with dimethylaminoacetaldehyde dimethylacetal is about 4-12 equivalents.

In some embodiments, in the method for preparing a compound SGL1-B, dimethylaminoacetaldehyde dimethylacetal (DMA-DMA) and hydrazine acetate are subjected to a one-pot process to efficiently configure a 5-membered heterocycle.

In some embodiments, the method for preparing a compound SGL1-B of the present disclosure can reach 10 Kg level scale-up yield of more than 70%.

In still another aspect, the present disclosure relates to a method for preparing a compound SGL1-C, comprising: subjecting a compound SGL1-B to a hydrolysis reaction under an alkaline condition.

In some embodiments, an addition amount of a base is about 3-10 equivalents based on the compound SGL1-B.

In some embodiments, illustrative examples of the alkaline condition that can be used in the method for preparing a compound SGL1-C of the present disclosure include, but are not limited to, sodium hydroxide, potassium hydroxide, lithium hydroxide, potassium carbonate, cesium carbonate, sodium carbonate, or any mixture thereof.

In some embodiments, the compound SGL1-B is subjected to a hydrolysis reaction in the presence of sodium hydroxide.

In some embodiments, the hydrolysis reaction is carried out at about 20-30°C.

In some embodiments, the hydrolysis reaction is carried out for about 3-20 h.

In some embodiments, the method for preparing a compound SGL1-C of the present disclosure can reach 10 Kg level scale-up yield of more than 70%.

In yet another aspect, the present disclosure relates to a method for preparing a compound SGL1, comprising: subjecting a compound SGL1-C to a reaction with a compound SGL1-SM2.

In some embodiments, the compound SGL1-C is subjected to the reaction with the compound SGL1-SM2 in the presence of a condensation agent.

In some embodiments, illustrative examples of the condensation agent that can be used in the reaction of the compound SGL1-C with the compound SGL1-SM2 include, but are not limited to, 1-ethyl-(3-dimethylaminopropyl)carbodiimide hydrochloride (EDCI), 2-(7-azabenzotriazole)-N,N,N',N'-tetramethylurea hexafluorophosphate (HATU), dicyclohexylcarbodiimide (DCC) , N,N'-diisopropylcarbodiimide (DIC), 1-hydroxybenzotriazole (HOBT), propylphosphonic anhydride (T₃P), n-butylphosphoric anhydride (T₄P), or any mixture thereof.

In some embodiments, the reaction of the compound SGL1-C with the compound SGL1-SM2 is carried out in the presence of n-butylphosphoric anhydride (T₄P).

In some embodiments, in the reaction of the compound SGL1-C with the compound SGL1-SM2, an addition amount of n-butylphosphoric anhydride (T₄P) is at least about 1.5 equivalents based on the compound SGL1-C.

In some embodiments, the compound SGL1-C is subjected to the reaction with the compound SGL1-SM2 under an alkaline condition.

In some embodiments, an addition amount of a base is at least 5 equivalents based on the compound SGL1-C.

In some embodiments, illustrative examples of the alkaline condition that can be used in the method for preparing a compound SGL1 of the present disclosure include, but are not limited to, N,N-diisopropylethylamine (DIEA), triethylamine (TEA), pyridine (Py), 4-dimethylaminopyridine (DMAP), 2,6-lutidine, N-methylmorpholine (NMM), potassium carbonate, sodium carbonate, or any mixture thereof.

In some embodiments, the compound SGL1-C is subjected to the reaction with the compound SGL1-SM2 in the presence of diisopropylethylamine (DIEA).

In some embodiments, an addition amount of the compound SGL1-SM2 is about 1.05-5 equivalents based on the compound SGL1-C.

In some embodiments, the compound SGL1-C is subjected to the reaction with the compound SGL1-SM2 at 20-30°C.

In some embodiments, the compound SGL1-C is subjected to the reaction with the compound SGL1-SM2 for about 5-20 h.

In some embodiments, the method for preparing a compound SGL1 of the present disclosure comprises dehydration condensation in the presence of n-butylphosphoric anhydride (T₄P), to efficiently complete the preparation of an amide, with the yield reaching more than 85%.

In another aspect, the present disclosure relates to a method for preparing a compound SGL1, comprising:
the method for preparing a compound SGL1-A according to the present disclosure; and/or
the method for preparing a compound SGL1-B according to the present disclosure; and/or
the method for preparing a compound SGL1-C according to the present disclosure; and/or
the method for preparing a compound SGL1 according to the present disclosure.

In still another aspect, the present disclosure relates to a method for preparing a compound SGL1, comprising:
subjecting a compound SGL1-SM1 to a hydrolysis reaction under an alkaline condition to obtain a compound SGL1-A;
subjecting the compound SGL1-A to a reaction with dimethylaminoacetaldehyde dimethylacetal and hydrazine acetate to obtain a compound SGL1-B;
subjecting the compound SGL1-B to a hydrolysis reaction under an alkaline condition to obtain a compound SGL1-C; and
subjecting the compound SGL1-C to a reaction with a compound SGL1-SM2 to obtain the compound SGL1.

In some embodiments, a method for preparing a heterocyclic regulator for lipid synthesis of the present disclosure can improve the yield, reduce impurities, and is adapted to industrial application.

The present disclosure will be explained in detail below with reference to the embodiments below to better understand various aspects of the present application and advantages thereof. However, it should be understood that the embodiments below are non-restrictive and are merely used to illustrate some embodiments of the present disclosure.

### Examples

The reagents and devices used in the examples of the present disclosure are all conventional and commercially available.

For example:

| Material | Manufacturer | CAS No. |
|---|---|---|
| Sodium hydroxide | Tianjin Pengkun Chemical Co., Ltd. | 1310-73-2 |
| 30% hydrogen peroxide | Shanghai Vokai Biotechnology Co., Ltd. | 7722-84-1 |
| Sodium bisulfite | Yonghua Chemical Co., Ltd. | 7631-90-5 |
| Hydrochloric acid | Hangzhou Electrochemical Group Co., Ltd. | 7647-01-0 |
| Tetrahydrofuran | Nantong Xingchen Synthetic Material Co., Ltd. | 109-99-9 |
| Dimethylaminoacetaldehyde dimethylacetal | Hubei Chenfang Pharmaceutical Chemical Co., Ltd. | 18871-66-4 |
| Hydrazine acetate | Suzhou Baker Biotechnology Co., Ltd. | 7335-65-1 |
| Acetic acid | Yonghua Chemical Co., Ltd. | 64-19-7 |
| Methanol | Anhui Huayi Chemical Co., Ltd. | 67-56-1 |
| Dichloromethane | Jiangsu Li & Man Chemical Ltd. | 75-09-2 |
| N,N-diisopropylethylamine | Zhejiang Pukang Chemical Co., Ltd. | 7087-68-5 |
| Solution of 50% n-butylphosphoric anhydride in ethyl acetate | Suzhou Highfine Biotech Co., Ltd. | 163755-62-2 |
| Anhydrous ethanol | Wujiang Yongxiang Alcohol Manufacturing Co., Ltd. | 64-17-5 |

| Device name | Tag No. | Capacity | Material |
|---|---|---|---|
| Reactor | R12001 | 500 L | Glass lining *GL* |
| Reactor | R12006 | 250 L | Glass lining *GL* |
| Reactor | R12003 | 300 L | Glass lining *GL* |
| Centrifuge | M11007 | DN1000 | *Hastelloy* |
| Centrifuge | M12002 | DN450 | Stainless steel *SST* |
| Dryer | D12001 | 350 L | Stainless steel *SST* |
| Dryer | D12002 | 350 L | Stainless steel *SST* |

### Example 1

### Preparation of compound SGL1-A

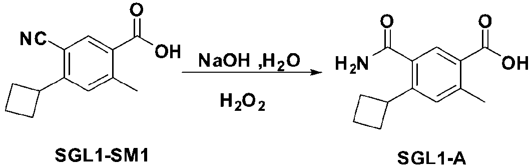

Purified water (6.03 equivalents, 66.33 Kg) was added into a reactor, stirring was started, and anhydrous ethanol (2.38 equivalents, 26.18 Kg) and sodium hydroxide (0.56 equivalents, 6.16 Kg) were added. After the mixture was fully stirred, SGL1-SM1 (1 equivalent, 11 Kg) was added, and the reactor was rinsed with purified water (1.05 equivalents, 11.55 Kg). An aqueous solution of 30% hydrogen peroxide (3.73, 41.03 Kg) was slowly added dropwise. After the addition was completed, the mixture was stirred at a controlled temperature of 25±5°C for 5 h, then sampled, and sent to HPLC for IPC-1 testing, until the reaction was up to standard. A solution of 20% sodium bisulfite (2.9 equivalents, 31.9 Kg) in water (11.5 equivalents, 126.5 Kg) was pre-prepared, and after the solution was up to standard, a 20% sodium bisulfite solution was added dropwise into the reactor. The mixture was stirred at 25±10°C for 1 h, and adjusted with hydrochloric acid to a pH=1-2. The mixture was stirred at a controlled temperature of 25±10°C for 2 h, and filtered. The filter cake was rinsed with purified water (3.04 equivalents, 33.44 Kg) to obtain a crude wet product of SGL1-A. Purified water (19.04 equivalents, 209.44 Kg) was added into the reactor, stirring was started, the crude wet product of SGL1-A was added, and walls of the reactor were washed with purified water (1.00 equivalents, 11 Kg). The mixture was heated to a temperature of 35±5°C, kept at the temperature for reaction for at least 1 h, cooled to a temperature of 20±5°C, and centrifuged. The filter cake was rinsed with purified water (3.0 equivalents, 33 Kg) twice to obtain a refined wet product of SGL1-A. The wet product was sampled and sent for IPC-2 analysis. The refined wet product of SGL1-A was transferred to a vacuum dryer with the dryer jacket controlled at a temperature of 55±5°C for drying for 15 h, and the dried product was sampled for IPC-3 testing of KF and LOD (KF≤0.5%, LOD≤2.0%). After the sample was up to standard, the dried product was cooled to collect powder, thus obtaining 11.09 Kg of the dry product of SGL1-A, with a yield of 93.0%.
¹H NMR (500 MHz, DMSO-d6) δ 12.37 (s, 1H),7.72 (s, 1H), 7.28 (d, J=7.8 Hz, 1H), 7.70 (d, J=7.8 Hz, 1H), 7.22 (s, 1H), 3.11 (q, J=5.3 Hz, 1H), 2.45 (s, 3H), 1.97-1.64 (m, 7H)

The above Example 1 was repeated using the following process parameters with the result as shown in Table 1.

**Table 1**

| Serial No. | SGL1-SM1 | NaOH | Hydrogen peroxide | Reaction temperature | Yield |
|---|---|---|---|---|---|
| 1 | 1.0 equivalent | 3.0 equivalents | 15.0 equivalents | 20-30°C | 90% |
| 2 | 1.0 equivalent | 3.0 equivalents | 7.0 equivalents | 20-30°C | 93% |
| 3 | 1.0 equivalent | 4.0 equivalents | - | 70-80°C | 65% |
| 4 | 1.0 equivalent | 2.0 equivalents | - | 70-80°C | 40% |

### Example 2

### Preparation of compound SGL1-B

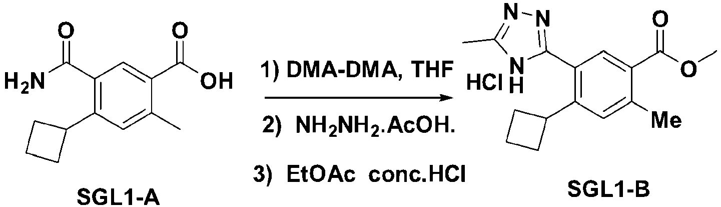

Tetrahydrofuran (6.27 equivalents, 68.97 Kg) was added into a reactor under nitrogen protection, and stirring was started. Dimethylaminoacetaldehyde dimethylacetal (DMA-DMA) (1.7173 equivalents, 18.8903 Kg) was added into the reactor. SGL1-A (1.0 equivalent, 11 Kg) was added into the reactor. After the addition was completed, walls of the reactor were washed with tetrahydrofuran (0.903 equivalents, 9.933 Kg). The system was heated to a temperature to 65±5°C, stirred at the temperature for 5 h, and sampled for IPC-1 testing. The in-process control was up to standard. The reactor was adjusted to a temperature of 30±10°C. Hydrazine acetate (0.596 equivalents, 6.556 Kg) was added into the reactor, and acetic acid (1.0724 equivalents, 11.7964 Kg) was added dropwise. After the dropwise addition was completed, the mixture was further stirred at the temperature of 30±10°C for additional 30 min, then heated to a temperature of 50±5°C, stirred at the temperature for 2 h, and sampled for IPC-2 testing.

After the reaction was completed, the mixture was cooled to a temperature of 30±10°C, purified water (7.245 equivalents, 79.695 Kg) was added into the reactor, and the mixture was concentrated under reduced pressure at a controlled temperature of ≤50°C to 7 times as much as the volume, cooled to a temperature of 20±5°C, stirred for 1 h, then centrifuged, and filtered. The filter cake was washed with purified water (2.092 equivalents, 23.012 Kg) to obtain SGL1-B in a free state. The wet product was transferred into a vacuum dryer with the jacket controlled at a temperature of 45-55°C for drying for at least 10 h, and sampled for IPC-3 testing of KF until KF≤2%, to obtain a dry product of SGL1-B in the free state. Under nitrogen protection, ethyl acetate (8.265 equivalents, 90.915 Kg) was added into the reactor, stirring was started; the dry product of SGL1-B in the free state was added, and after the addition was completed, walls of the reactor were washed with ethyl acetate (0.878 equivalents, 9.658 Kg). The mixture was stirred for 10 min, heated to a temperature of 60±5°C, stirred at the temperature for 1 h, and then cooled to a temperature of 25±5°C. The reactor was controlled at a temperature of 20±10°C, and hydrochloric acid (0.51 equivalents, 5.61 Kg) was added dropwise. The mixture was stirred at this temperature for 1 h, and centrifuged. The filter cake was washed with ethyl acetate (3.571 equivalents, 39.28 Kg) to obtain a wet product of SGL1-B. The wet product of SGL1-B was transferred to a vacuum dryer with the jacket controlled at a temperature of 45-55°C, for drying for at least 10 h, and sampled for IPC-5 testing of LOD until LOD≤2%. After the sample was up to standard, 10.87 Kg of the dry product of SGL1-B was obtained, with a yield of 71.6%.
¹H NMR (500 MHz, DMSO-d6) δ 11.05 (s, 2H), 8.49 (s, 2H), 7.34 (s, 2H), 3.88 (s, 6H), 3.29 (dd, J=5.6, 4.9 Hz, 2H), 2.78 (s, 6H), 2.50 (s, 6H), 1.96-1.87 (m, 4H), 1.87-1.81 (m, 1H), 1.81-1.76 (m, 5H), 1.75 (d, J=1.7 Hz, 1H), 1.74-1.64 (m, 2H)

The above Example 2 was repeated using the following process parameters with the result as shown in Table 2.

**Table 2**

| Serial No. | SGL1-A | DMA-DMA | Hydrazine acetate | Acetic acid | Reaction temperature | Yield |
|---|---|---|---|---|---|---|
| 1 | 1.0 equivalent | 3.0 equivalents | 1.5 equivalents | 4.0 equivalents | 60-70°C | 71.6% |
| 2 | 1.0 equivalent | 1.0 equivalent | 1.5 equivalents | 4.0 equivalents | 60-70°C | 10% |
| 3 | 1.0 equivalent | 2.0 equivalents | 1.5 equivalents | 4.0 equivalents | 60-70°C | 55% |
| 4 | 1.0 equivalent | 6.0 equivalents | 1.5 equivalents | 4.0 equivalents | 60-70°C | 72% |
| 5 | 1.0 equivalent | 3.0 equivalents | 1.5 equivalents | 7.0 equivalents | 60-70°C | 71% |
| 6 | 1.0 equivalent | 3.0 equivalents | 1.0 equivalent | 4.0 equivalents | 60-70°C | 40% |
| 7 | 1.0 equivalent | 3.0 equivalents | 3.0 equivalents | 4.0 equivalents | 60-70°C | 70% |
| 8 | 1.0 equivalent | 3.0 equivalents | 1.5 equivalents | 4.0 equivalents | 40-50°C | 30% |

### Example 3

### Preparation of compound SGL1-C

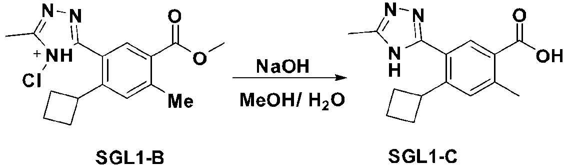

Under nitrogen protection, methanol (3.58 equivalents, 38.66 Kg) was added into a reactor, stirring was started, purified water (1.53 equivalents, 16.52 Kg) was added into the reactor, the reactor was controlled at a temperature of below 50°C, and sodium hydroxide (0.369 equivalents, 3.99 Kg) was added into the reactor. The mixture was stirred for at least 30 min, then SGL1-B (1.0 equivalent, 10.80 Kg) was added, and after the addition was completed, walls of the reactor were washed with methanol (0.82 equivalents, 8.86 Kg). Then, the system was heated to a temperature of 60±5°C, stirred at the temperature for 3 h, and sampled for IPC-1 testing, until SGL1-B≤0.5%. After the sample was up to standard, the reactor was cooled to a temperature of 40±5°C, purified water (4.77 equivalents, 51.52 Kg) was added into the reactor, and the mixture was concentrated under reduced pressure to 6 times as much as the volume. The reactor was cooled to a temperature of 20±10°C, and controlled at this temperature with dropwise addition of hydrochloric acid. The mixture was adjusted to a pH of 1-3, stirred at the temperature for 1 h, and centrifuged. The filter cake was rinsed with purified water (5.0 equivalents, 54 Kg) to obtain a wet product of SGL1-C. The wet product up to standard was transferred to a vacuum dryer with the jacket controlled at a temperature of 40-55°C, for drying for at least 10 h, and sampled for IPC-3 testing of KF until KF≤0.5%, thus obtaining 8.79 Kg of a dry product of SGL1-C, with a yield of 96.6%.
¹H NMR (500 MHz, DMSO-d6) δ 12.06 (s, 1H), 10.98 (s, 1H), 8.21 (s, 1H), 7.27 (s, 1H), 3.34 (dd, J=5.6, 4.9 Hz, 1H), 2.43 (d, J=16.1 Hz, 6H), 1.98-1.87 (m, 3H), 1.84 (dd, J=7.6,5.3 Hz, 1H), 1.84-1.78 (m,2H), 1.81-1.71 (m, 1H), 1.74-1.66 (m, 1H)

The above Example 3 was repeated using the following process parameters with the result as shown in Table 3.

**Table 3**

| Serial No. | SGL1-B | NaOH | Reaction temperature | Reaction time | Yield |
|---|---|---|---|---|---|
| 1 | 1.0 equivalent | 3.0 equivalents | 20-30°C | 3 h | 98% |
| 2 | 1.0 equivalent | 2.0 equivalents | 20-30°C | 3 h | 93% |

### Example 4

### Preparation of compound SGL1

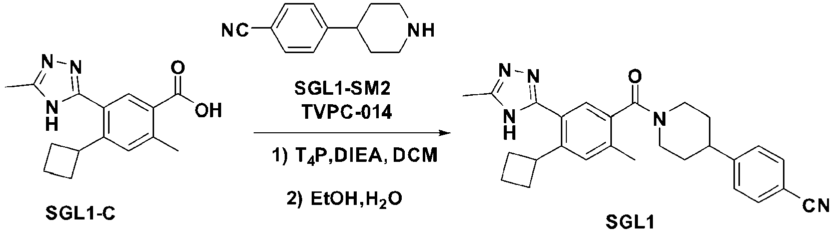

Dichloromethane (6.88 equivalents, 60.54 Kg) was added into a reactor under nitrogen protection, and stirring was started. SGL1-C (1.0 equivalent, 8.8 Kg) and SGL1-SM2 (0.86 equivalents, 7.6 Kg) were added into the reactor; and after the addition was completed, walls of the reactor were washed with dichloromethane (1.34 equivalents, 11.79 Kg); the reactor was adjusted to a temperature of 15±5°C, N,N-diisopropylethylamine (2.36 equivalents, 20.77 Kg) was added dropwise, and a solution of 50% butylphosphoric anhydride in ethyl acetate (4 equivalents, 35.2 Kg) was added dropwise. After the dropwise addition was completed, the mixture was adjusted to room temperature for reaction for 2 h, and then sampled for IPC-1 testing, until SGL1-C≤2%. After the sample was up to standard, ethyl acetate (9.03 equivalents, 79.46 Kg) was added into the reactor, the mixture was concentrated under reduced pressure at 40°C to 15 times as much as the volume, and purified water (5.06 equivalents, 44.53 Kg) was added. The mixture was stirred for 2 h, and centrifuged. The filter cake was washed with purified water (1.99 equivalents, 17.51 Kg) to obtain a wet product 1 of SGL1. Anhydrous ethanol (2.73 equivalents, 24.02 Kg) was added into the reactor, stirring was started, the wet product 1 of SGL1 was added, the reactor was heated to a temperature of 78±5°C, and the mixture entered a clean region through a precision filter at this temperature. After the filtration was completed, purified water (1.74 equivalents, 15.31 Kg) was added dropwise, and then the mixture was further stirred at this temperature at least for additional 0.5 h. The system was cooled to a temperature of 15±10°C within 6 h, stirred at this temperature for 6 h, and centrifuged. The filter cake was washed with a mixed solution of ethanol (0.47 equivalents, 4.14 Kg) and purified water (0.47 equivalents, 4.14 Kg) at 1:1 to obtain a refined wet product of SGL1, and sampled for IPC-2 testing; the refined wet product of SGL1 was dried under reduced pressure at 40-55°C for 10 h, and sampled for IPC-3 testing of residual solvent by GC, until the sample was up to standard, thus obtaining 13.085 Kg of SGL1, with a yield of 88.1%.

¹HNMR (400 MHz, DMSO-d6) δ 13.66 (s, 1H), 7.75 (d, J=8.0 Hz, 2H), 7.55 (s, 1H), 7.47 (d, J=8.0 Hz, 2H), 7.37 (s, 1H), 4.72 (d, *J*=12.5 Hz, 1H), 4.31 (s, 1H), 3.44 (m, 1H), 3.13 (*t, J*=12.0 Hz, 1H), 2.90-2.85 (m, 2H), 2.39 (s, 3H), 2.36-2.26 (m, 3H), 2.17 (s, 2H), 2.07-2.00 (m, 2H), 1.89-1.71 (m, 3H), 1.67-1.64 (m, 2H),1.46 (s, 1H).

The above Example 4 was repeated using the following process parameters with the result as shown in Table 4.

**Table 4**

| Serial No. | SGL1-C | T₄P | DIEA | SGL1-SM2 | Reaction temperature | Reaction time | Yield |
|---|---|---|---|---|---|---|---|
| 1 | 1.0 equivalent | 1.5 equivalents | 5.0 equivalents | 1.05 equivalents | 20-30°C | 5 h | 88% |
| 2 | 1.0 equivalent | 1.1 equivalents | 5.0 equivalents | 1.05 equivalents | 20-30°C | 5 h | 65% |
| 3 | 1.0 equivalent | 1.3 equivalents | 5.0 equivalents | 1.05 equivalents | 20-30°C | 5 h | 78% |
| 4 | 1.0 equivalent | 1.5 equivalents | 3.0 equivalents | 1.05 equivalents | 20-30°C | 5 h | 60% |

In the present disclosure, relational terms such as "first" and "second" are only used to distinguish one entity or operation from another entity or operation, and do not necessarily require or imply existence of any actual relationship or sequence between these entities or operations.

As can be understood from the above description, although specific embodiments of the present disclosure have been described for illustrative purposes, various modifications or improvements may be made by those skilled in the art without departing from the spirit and scope of the present disclosure. These modifications or improvements should all be encompassed within the scope of the appended claims of the present disclosure.

## Claims

1. A method for preparing a compound SGL1-A, comprising: subjecting a compound SGL1-SM1 to a hydrolysis reaction under an alkaline condition, wherein an aqueous solution of hydrogen peroxide is added to the hydrolysis reaction.

2. The method according to claim 1, wherein an addition amount of the aqueous solution of hydrogen peroxide is 7-15 equivalents based on the compound SGL1-SM1.

3. The method according to claim 1 or 2, wherein the hydrolysis reaction is carried out at 20-30°C.

4. A method for preparing a compound SGL1-B, comprising: subjecting a compound SGL1-A to a reaction with dimethylaminoacetaldehyde dimethylacetal and hydrazine acetate.

5. The method according to claim 4, wherein an addition amount of dimethylaminoacetaldehyde dimethylacetal is 3.0-6.0 equivalents based on the compound SGL1-A.

6. The method according to claim 4 or 5, wherein an addition amount of hydrazine acetate is 1.5-3.0 equivalents based on the compound SGL1-A.

7. The method according to any one of claims 4 to 6, wherein the reaction is carried out at 60-70°C.

8. The method according to any one of claims 4 to 7, wherein the method further comprises refining a product of the reaction of the compound SGL1-A with dimethylaminoacetaldehyde dimethylacetal and hydrazine acetate using an organic solvent, preferably methanol, ethanol, isopropanol, methyl tert-butyl ether, n-heptane, isopropyl acetate, acetone, methyl isobutyl ketone, isobutyl acetate, ethyl acetate or any mixture thereof, more preferably ethyl acetate.

9. The method according to any one of claims 4 to 8, wherein the method further comprises adding an acid, preferably formic acid, acetic acid, phosphoric acid, hydrochloric acid, maleic acid, fumaric acid or any mixture thereof, more preferably acetic acid, to the reaction of the compound SGL1-A with dimethylaminoacetaldehyde dimethylacetal and hydrazine acetate.

10. A method for preparing a compound SGL1-C, comprising: subjecting a compound SGL1-B to a hydrolysis reaction under an alkaline condition.

11. The method according to claim 10, wherein an addition amount of a base is 3-10 equivalents based on the compound SGL1-B.

12. The method according to claim 10 or 11, wherein the alkaline condition is selected from sodium hydroxide, potassium hydroxide, lithium hydroxide, potassium carbonate, cesium carbonate, sodium carbonate, or any mixture thereof, preferably sodium hydroxide.

13. The method according to any one of claims 10 to 12, wherein the hydrolysis reaction is carried out at 20-30°C.

14. The method according to any one of claims 10 to 13, wherein the hydrolysis reaction is carried out for 3-20 hs.

15. A method for preparing a compound SGL1, comprising: subjecting a compound SGL1-C to a reaction with a compound SGL1-SM2.

16. The method according to claim 15, wherein the reaction is carried out in the presence of a condensation agent, preferably the condensation agent is selected from 1-ethyl-(3-dimethylaminopropyl)carbodiimide hydrochloride (EDCI), 2-(7-azabenzotriazole)-N,N,N',N'-tetramethylurea hexafluorophosphate (HATU), dicyclohexylcarbodiimide (DCC), N,N'-diisopropylcarbodiimide (DIC), 1-hydroxybenzotriazole (HOBT), propylphosphonic anhydride (T₃P), n-butylphosphoric anhydride (T₄P), or any mixture thereof, more preferably n-butylphosphoric anhydride (T₄P), and even more preferably an addition amount of n-butylphosphoric anhydride (T₄P) is at least 1.5 equivalents based on the compound SGL1-C.

17. The method according to claim 15 or 16, wherein the reaction is carried out under an alkaline condition, and preferably an addition amount of a base is at least 5 equivalents based on the compound SGL1-C.

18. The method according to claim 17, wherein the alkaline condition is selected from N,N-diisopropylethylamine (DIEA), triethylamine (TEA), pyridine (Py), 4-dimethylaminopyridine (DMAP), 2,6-dimethylpyridine, N-methylmorpholine (NMM), potassium carbonate, sodium carbonate, or any mixture thereof, preferably diisopropylethylamine (DIEA).

19. A method for preparing a compound SGL1, comprising:
the method according to any one of claims 1 to 3; and/or
the method according to any one of claims 4 to 9; and/or
the method according to any one of claims 10 to 14; and/or
the method according to any one of claims 15 to 18.

20. A method for preparing a compound SGL1, comprising:
subjecting a compound SGL1-SM1 to a hydrolysis reaction under an alkaline condition to obtain a compound SGL1-A;
subjecting the compound SGL1-A to a reaction with dimethylaminoacetaldehyde dimethylacetal and hydrazine acetate to obtain a compound SGL1-B;
subjecting the compound SGL1-B to a hydrolysis reaction under an alkaline condition to obtain a compound SGL1-C; and
subjecting the compound SGL1-C to a reaction with a compound SGL1-SM2 to obtain the compound SGL1.
